# EUROPEAN PATENT APPLICATION

(11) **EP 2 851 824 A1**
(43) Date of publication of application: **25.03.2015**
(21) Application number: 13185398.8
(22) Date of filing: 20.09.2013
(51) Int. Cl.: G06F 19/00

(54) **Data management unit for supporting health control**

(71) Applicant: Sanofi-Aventis Deutschland GmbH, 65929 Frankfurt am Main (DE)
(72) Inventor: The designation of the inventor has not yet been filed

(57) **Abstract**

The present invention refers to a data management unit for supporting health control comprising
• a display (162),
• a processor (140) adapted to receive a user-selectable group of measurement values of a plurality of measurement values of a physiological parameter, for example a blood glucose level, to calculate at least one derived value (501 a, 503a, 506a) from the group of measurement values and to determine for each derived value (501 a, 503a, 506a) a corresponding rating value and / or a corresponding trend value,
• a trend indicator (164), wherein the trend indicator is adapted to indicate different trend categories on the display (162), for example an increasing trend, a steady trend and a decreasing trend, as a respective graphical picture (503d),
• a color indicator (166), wherein the color indicator is adapted to indicate an area (501 b, 503b, 506b) with at least one of two different colors on the display (162), for example a red color and a green color,

wherein the display (162) is adapted to display the at least one derived value (501 a, 503a, 506a), each in a separate tile (501, 502, 503, 504, 505, 506, 507), as a numerical value and / or a graphical picture,
wherein the processor (140) interacts with the trend indicator (164) such that the trend indicator (164) displays within each tile (501, 502, 503, 504, 505, 506, 507) one of the different trend categories based at least in part on the corresponding trend value of the at least one derived value (501 a, 503a, 506a) of the respective tile (501, 502, 503, 504, 505, 506, 507) and / or
the processor (140) interacts with the color indicator (166) such that the color indicator displays within each tile one area (501 b, 503b, 506b) of one of the different colors based at least in part
• on the at least one derived value (501 a, 503a, 506a) of the respective tile (501, 502, 503, 504, 505, 506, 507) and / or
• on the corresponding rating value of the at least one derived value (501 a, 503a, 506a) of the respective tile (501, 502, 503, 504, 505, 506, 507) and / or

on the corresponding trend value of the at least one derived value (501 a, 503a, 506a) of the respective tile (501, 502, 503, 504, 505, 506, 507). Additionally, the invention refers to a corresponding method and a computer program for health control, and to a corresponding computer program product.

## Description

The present invention relates to a data management unit for supporting health control, a method and a computer program for supporting health control, a respective computer program product, and a medical device for supporting health control.

The following description of the invention mainly refers to diabetes as a health problem and the blood glucose level as the physiological parameter to be controlled in order to assess the effectiveness of the prescribed treatment. However, the invention may also be used with regard to other health problems and for management of other physiological parameter data like (a) blood pressure in hypertensive heart disease, (b) cholesterol or lipoprotein profile in patients with risk factors for heart disease and stroke, (c) peak flow in asthmatic patients, or (d) coagulation in patients treated for hemophilia.

Diabetes mellitus is a group of metabolic diseases in which a person has high blood sugar, either because the pancreas does not produce enough insulin, or because cells do not respond to the insulin that is produced. The treatment of diabetes concentrates on keeping blood sugar levels as close to normal ("euglycemia") as possible, without causing hypoglycemia. This can usually be accomplished with diet, exercise, and use of appropriate medications (insulin in the case of type 1 diabetes; oral medications, as well as possibly insulin, in type 2 diabetes).

Essential elements of the management of diabetes with insulin are periodic checks of the glucose concentration in the blood performed by the patients themselves, in order to obtain regular information on the progress and success of the prescribed treatment. This understanding, and patient participation is vital, since the complications of diabetes are far less common and less severe in people who have well-managed blood sugar levels. With regard to this it has to be considered that the blood glucose level fluctuates throughout the day and is directly influenced by the amount of insulin administered, as well as lifestyle factors such as the amount and kind of food that is consumed, the exercise level and stress.

Therefore, the monitoring of the sugar level in the blood serves a dual purpose: on the one hand it provides the patient with information about the current status of glycemic control. On the other hand can the measured values serve as information for the patient or a healthcare professional (HCP) to determine whether an adjustment in the medication, namely the amount of insulin to be taken, is indicated.

In order to achieve these goals or to get as close as possible to the desired glycemic control, it is common practice that blood glucose measurement (BGM) values are monitored once or several times during the day, following a testing regime normally prescribed by an HCP.

A special role is played by the so-called fasting blood glucose measurement value (FBG). A fasting blood glucose value is derived after several hours without eating (6 to 8 hours). The fasting blood glucose measurement value is typically taken in the morning before breakfast and is the most commonly performed test among insulin treated patients as it is used to assess the quality of the titration of long-acting basal insulin or analogs such as insulin glargine.

In order to adjust or to adapt the therapy it is helpful to record the results of all blood glucose measurements and to analyze these results with a data management unit. Therefore, typically a portable monitor is used which may be able to generate and store blood glucse measurement values in an internal data storage or which receives the measurement values from a blood glucose measurement device, i.e. from an external data storage. A wireless or wired data transfer can be used to transport the results from the measurement device to the data management unit.

Data management units of prior art usually store the measurement values and are typically able to show graphical representation of data and statistical measures such as average values, which are often difficult for a user to interpret and which are not or not very suitable to make decisions on the improvement of the treatment. The existing data displays typically require an experienced user and further analysis of the results, which is time consuming. Further it is observed that a single self-monitoring blood glucose measurement value may be interpreted differently if it is seen in context of previous measurement values of the same time range.

Common devices of prior art allow the individual test result to be associated by the user with a tag, intended to indicate a result taken as before or after a meal for more detailed analysis.

For example for morning fasting readings and the purpose of determining the quality of the titration of long-acting insulin though, it is important to detect trends over a certain period of time as common treatment algorithms for long-acting insulin rely mainly on morning fasting values. Therefore, the need exists to provide a data management unit which provides a data management that is easy to understand for the patient and useful to the HCP when adjusting the prescribed therapy.

Documents US 7,904,310 B2 and US 7,853,455 B2 disclose a system and method that enables a health care provider to monitor and manage a health condition of a patient wherein the user can select graphic display of blood glucose test results over a specific period of time such as a particular week. When such a weekly trend graph is displayed, small icons identify points on the graphic representation that correspond to the blood glucose test results, wherein coordinate values for blood glucose level and measurement time of day can be displayed if desired. Further, the graph consists of lines interconnecting points that correspond to the blood glucose test results and the average blood glucose level as well as the standard deviation of the measurement values may be displayed. For people without higher mathematical education the interpretation of such a graph, the average value and the standard deviation is too difficult.

Documents WO 2010/149392 A1 and EP 2 486 851 A1 disclose a method and system for providing an estimated true mean blood glucose value from spot blood glucose measurements. This system comprises a display, memory and a processor program to collect spot blood glucose measurement values and associated context of the blood glucose measurements at daily times and events specified by a structured sampling schema provided in memory, to weight each of the connected blood glucose measurement values based on the associated context and to determine the estimated true mean blood glucose value from the weighted measurements of the collected blood glucose measurements and to provide the estimated true mean blood glucose value to the display. Any trend information cannot be derived from these true mean blood glucose values.

Documents WO 03/057027 A2 and EP 2 259 057 A1 refer to a system for monitoring physiological characteristics according to the user biological state comprising a sensor input capable of receiving a signal from a sensor, the signal being based on a sensed physiological characteristic value of a user, a memory for storing a plurality of measurements of the sensed physiological characteristic value of the user from the received signal from the sensor and a display for presenting a graphical representation of the plurality of measurements of the sensed physiological characteristic value preferably continuously in real time. Further, the graphical representation may comprise one or more trend indicators indicating an approximate rate trend in the sensed physiological characteristic value over a recent series of the plurality of measurements. Therefore, different trend values are defined, for example a high positive trend threshold which defines a limit at 3 mg/dl per minute averaged over 20 minutes. A low positive trend threshold defines a limit at 1 mg/dl per minute, a high negative trend threshold a limit at -3 mg/dl per minute and a low negative trend threshold a limit at -1 mg/dl per minute, each averaged over 20 minutes. This prior art method of trend representation is only useful for continuous measurement of blood glucose level.

Document EP 2 031 534 A1 refers to a diabetes management system and process that may be used to analyze and recognize pattern for a large number of blood glucose concentration measurements and other physiological parameters related to the glycemia of a patient. It is disclosed that a display area includes a graphical pattern of blood glucose variability about a median blood glucose value by at least one of time of day, of day in a week, of both time of day and day of week, or at different predetermined intervals. A diagram shows a graphical blood glucose variability pattern in relation to a specific day as spanning from 24 hours starting at about 12 a.m. to about 12 a.m. of the next day. Therein, the median of glucose concentration values and a median of test times during this temporal period are shown defining a data point on a graph depicted by a bold line. As one can derive from the respective diagram such a trend representation is rather difficult to interpret by the patient.

Hence, the object of the present invention is to provide a data management unit and a respective method which avoids the above drawbacks and provides information for self-monitoring of a physiological parameter containing reliable trend information which is easy to understand by the patient and useful to the HCP when adjusting the prescribed therapy.

The above problem is solved by a data management unit with the features of claim 1.

In particular the inventive data management unit for supporting health control comprises
- a display,
- a processor adapted to receive a user-selectable group of measurement values of a plurality of measurement values of a physiological parameter, for example a blood glucose level, to calculate at least one derived value from the group of measurement values and to determine for each derived value a corresponding rating value and / or a corresponding trend value,
- a trend indicator, wherein the trend indicator is adapted to indicate different trend categories on the display, for example an increasing trend, a steady trend and a decreasing trend, as a respective graphical picture,
- a color indicator, wherein the color indicator is adapted to indicate an area with one of at least two different colors on the display, for example a red color and a green color,
wherein the display is adapted to display the at least one derived value, each in a separate tile, as a numerical value and / or a graphical picture,
wherein the processor interacts with the trend indicator such that the trend indicator displays within each tile one of the different trend categories based at least in part on the corresponding trend value of the at least one derived value of the respective tile and / or the processor interacts with the color indicator such that the color indicator displays within each tile one area of one of the at least two different colors based at least in part
- on the at least one derived value of the respective tile and / or
- on the corresponding rating value of the at least one derived value of the respective tile and / or
- on the corresponding trend value of the at least one derived value of the respective tile.

The inventive data management unit enables a health control which is easy to understand and does not need higher mathematical education. In particular, the patient/user is provided with an easy understandable overview over the main parameters with regard to the disease so that a decision for health control may be reached easier and / or faster by the patient or HCP.

Each tile shown at the display provides one derived value calculated from a user-selectable group of measurement values of the plurality of measurement values, e.g. from the blood glucose measurement values of one or two weeks or a month, a trend category of a corresponding trend value and / or one area of one of the at least two different colors based on the derived value and / or the corresponding rating value and / or the corresponding trend value. Usually several tiles are displayed, wherein the order of the tiles may be defined by the user, also whether one particular tile is visible or not, or the order of the tiles may be generated by the data management unit automatically dependent on the relevance or criticality of patient status. E.g. the tiles with critical information of the patient may always stay on top of the tiles list.

Hence, the tile comprises a combination of color coding and a numerical value or graphic picture. Such a tile has for example a rectangular form but may have alternatively a triangular, quadratic, pentagonal, hexagonal or circular or any other suitable form. The area of color may have a rectangular or bar-like form accommodated on one side of the tile, may have the same form as the tile and forming the background of the tile or may form a frame of the tile. Other forms of the colored area are possible as well.

In one embodiment each tile may be flipped over e.g. by single or double clicking on the tile so that settings for the respective tile and / or derived value can be viewed or changed, for example the displayed derived value with its ranges or thresholds may be defined, the unit of the derived value or settings for the deduced rating value or trend value and the respective colors and graphical symbol pictures may be chosen and confirmed.

According to the invention, one derived value is for example a mean value of the user-selectable group of measurement values, for example an average fasting blood glucose value over the last 2 weeks, an average carbohydrate value per day or an average insulin value per day over a time period of 2 weeks, or a distribution of the selected group of measurement values within pre-defined intervals, for example a diagram showing the distribution of blood glucose measurement values which are within a target area, below the target area or above the target area, a number of particular, pre-defined measurement values within the group of measurement values, e.g. the number of hypos (hypoglycemic blood glucose values) or hypers (hyperglycemic blood glucose values) within the selected group, or an estimated value which is calculated using measured blood glucose values, for example an estimated HbA1 c value, or any other suitable value supporting health control which may be derived by calculation from the stored measurement values of the physiological parameter. Additionally, a laboratory value not deduced from the selected group of measurement values but separately determined for example in a laboratory, e.g. provided by the user / patient or the HCP, may be displayed for comparison in a separate tile.

The rating value is a value that describes the assessment of the respective derived value. For example, if the mean blood glucose value is within target, for example between 70 and 160 mg / dl, the rating value yields 1, wherein, if the mean blood glucose value is out of target, the rating value yields 0. From the rating value for example the color of the colored area may be determined by the color indicator directly, e.g. a rating value = 1 results in a green color and a rating value = 0 results in a red color. The color indicator may also represent a rating value in which a third rating / color is provided by defining e.g. a third range for the mean blood glucose value between an "in target" range and "out of target" range. In an example embodiment, the "out of target" range is below 50 mg / dl or above 180 mg / dl. The "in target" range is between 80 mg / dl and 140 mg / dl. The third range is then between 50 and 80 mg / dl and between 140 and 180 mg / dl. For example, if the glucose value is in the "in target" range, the rating value yields 1. If the mean blood glucose value is in the third range, the rating value yields 0.5, and if the mean blood glucose level is out of the "in target" and out of the third range, i.e. "out of target", the rating value yields 0. From the rating value the color of the colored area may be determined by equating a rating of 1 with a green color, of 0.5 with a yellow color and of 0 with a red color.

In an alternative embodiment, no number rating is provided, but a color is directly assigned. For example, for a glucose value below 50 mg / dl or above 180 mg / dl a red color is assigned. For a glucose value between 50 and 80 mg / dl or between 140 and 180 mg / dl a yellow color is assigned, and for a glucose value between 80 and 140 mg / dl a green color is assigned.

The plurality of measurement values of the physiological parameter may be received from a data storage internal or external of the data management unit. In an embodiment the plurality of measurement values of the respective user / patient or a plurality of users/patients is stored in a data storage of a cloud network infrastructure and may be downloaded from there for calculation and / or for usage in a tile of the display. In this embodiment the data management unit provides a respective web interface for both the user / patient and the HCP.

Accordingly, the above problem is solved by a medical device for supporting health control, the device comprising the above explained data management unit.

For the same reason the above problem is also solved by a method for supporting health control for a data management unit comprising the following steps:
- receiving a user-selectable group of measurement values of a plurality of measurement values of a physiological parameter, for example a blood glucose level,
- calculating at least one derived value from the group of measurement values and determining for each derived value a corresponding rating value and / or a corresponding trend value,
- displaying on a display the at least one derived value, each in a separate tile, as a numerical value and / or a graphical picture,
- indicating within each tile one of different trend categories, for example an increasing trend, a steady trend and a decreasing trend, as a respective visible graphical picture, based at least in part on the corresponding trend value of the at least one derived value of the respective tile, and / or
- indicating within each tile one area with one of at least two different colors, for example a red color and a green color, based at least in part
   ○ on the at least one derived value of the respective tile and / or
   ○ on the corresponding rating value of the at least one derived value of the respective tile and / or
   ○ on the corresponding trend value of the at least one derived value of the respective tile.

In another embodiment the data management unit further comprises a symbol indicator, wherein the symbol indicator is adapted to indicate one of different graphical symbol pictures on the display, for example an exclamation mark, a minus mark or a check mark symbol, wherein the processor interacts with the symbol indicator such that the symbol indicator displays within each tile one of the different graphical symbol pictures based at least in part
- on the at least one derived value of the respective tile and / or
- on the corresponding rating value of the at least one derived value of the respective tile and / or
- on the corresponding trend value of the at least one derived value of the respective tile.

Preferably, the tile may show the graphical symbol picture as information in addition to the displayed trend category and the colored area so that the status of the health control may be assessed even better.

In a preferred embodiment the graphical symbol picture has the same meaning as the color of the colored area, so that the graphical symbol picture corresponds to the color of the colored area and vice versa. In this case, the content of the color coding is also visible for color-blind user. Preferably, the colored area and the graphical symbol picture are combined such that the symbol indicator shows the graphical symbol picture within the colored area provided by the color indicator.

With analogous advantages the inventive method comprises the additional step of indicating within each tile one of different graphical symbol pictures, for example an exclamation mark, a minus mark or a check mark, based at least in part
○ on the at least one derived value of the respective tile and / or
○ on the corresponding rating value of the at least one derived value of the respective tile and / or
○ on the corresponding trend value of the at least one derived value of the respective tile.

In a further embodiment the data management unit comprises a first matrix referring to different ranges of the at least one derived value and / or to different ranges of the corresponding rating values and / or to different ranges of the corresponding trend values, wherein the first matrix assigns one of the at least two different colors to each range of aforementioned values or to each combination of ranges of aforementioned values. This means that the first matrix either defines particular ranges of the derived value or trend value or rate value and assigns each range to a particular color, for example red color, yellow color and green color, or it defines particular combination of ranges of at least two of the mentioned values and assigns each combination to a particular color, e.g. a mean blood glucose value above target with a downward trend may be assigned to the green color, a mean blood glucose value within the target range with a downward trend may be assigned to the yellow color, and a blood glucose value below target with a downward trend may be assigned to the red color.

In an embodiment of the inventive method, wherein one of the at least two different colors is chosen from the above described first matrix.

In another embodiment, the data management unit comprises a second matrix referring to different ranges of the at least one derived value and / or to different ranges of the corresponding rating values and / or to different ranges of the corresponding trend values, wherein the second matrix assigns one of the different graphical symbol pictures to each range of aforementioned values or to each combination of ranges of aforementioned values. Analogous to the above described first matrix the second matrix defines particular ranges of the mentioned values or combinations of ranges of at least two of the mentioned values and assigns each range or combination of ranges, respectively, to a particular graphical symbol picture.

In an embodiment of the inventive method one of the different graphical symbol pictures is chosen from the above described second matrix.

Using above first and second matrices allows an easy assignment of the colors / graphical symbol pictures to the corresponding ranges of the values. This embodiment further makes it possible to change the range thresholds by the user / patient and / or the HCP.

In another embodiment of the data management unit or method for health control the trend indicator is adapted to show on the display an up arrow as the increasing trend sign, a down arrow as the decreasing trend sign and a horizontal arrow as the steady trend sign. These signs are easy to understand for the user / patient.

In another embodiment of the data management unit or method, the data storage is further adapted to consider an associated event tag for user selection of the group of measurement values of the plurality of measurement values and / or for calculation of the derived value from the group of measurement values for each measurement value. For example blood glucose measurement values could be categorized as fasting blood glucose measurement value, as a pre-meal blood glucose measurement value or a post-meal blood glucose measurement value, or as a measurement value of a control solution or with a no-tag. By using such an event tag it can be guaranteed that only (the mean values of) the measurement values of the same category, e.g. the fasting blood glucose measurement values, are compared.

With the same advantages as explained above, the above problem is solved by a computer program for supporting health control comprising:
- code for receiving of a user-selectable group of measurement values of a plurality of measurement values of a physiological parameter, for example a blood glucose level,
- code for calculating at least one derived value from the group of measurement values and determining for each derived value a corresponding rating value and / or a corresponding trend value,
- code for indicating on a display the at least one derived value, each in a separate tile, as a numerical value and / or a graphical picture,
- code for indicating within each tile one of different trend categories, for example an increasing trend, a steady trend and a decreasing trend, as a respective visible graphical picture, based at least in part on the corresponding trend value of the at least one derived value of the respective tile and / or
- code for indicating within each tile an area with one of at least two different colors, for example a red color and a green color, based at least in part
   ○ on the at least one derived value of the respective tile and / or
   ○ on the corresponding rating value of the at least one derived value of the respective tile and / or
   ○ on the corresponding trend value of the at least one derived value of the respective tile.

In an embodiment and with the advantages as explained above the computer program further comprises code for indication within each tile one of different graphical symbol pictures, for example an exclamation mark and a check mark, based at least in part
○ on the at least one derived value of the respective tile and / or
○ on the corresponding rating value of the at least one derived value of the respective tile and / or
○ on the corresponding trend value of the at least one derived value of the respective tile.

The above computer program may be realized with the embodiments as mentioned above with regard to the inventive method for supporting health control.

The above problem is further solved by a computer program product comprising a computer-readable medium bearing computer program code embodied therein for use with a computer, wherein the computer program code comprises the above mentioned computer program.

The above-mentioned advantages as well as other advantages of various aspects of the present invention will become apparent to those of ordinary skill in the art by reading the following detailed description with the explanation of the accompanying drawings. All features described above and below and / or illustrated per se or in any combination form the subject-matter of the invention, independent of their inclusion in the claims or their back-reference. Exemplary embodiments of the present invention are described herein with reference to schematic drawings, in which
- Fig. 1: shows the medical device according to a preferred embodiment of the invention in a perspective view;
- Fig. 2: shows a diagram of the medical device as shown in Fig. 1;
- Fig. 3: shows a flow diagram containing a procedure realized by the inventive medical device in the "Measure BG" mode;
- Fig. 4: shows a first view of a display of the inventive medical device as depicted in Fig. 1;
- Fig. 5: shows another flow diagram comprising a part of the inventive method for supporting health control;
- Fig. 6: shows a second view of a display of the inventive medical device as depicted in Fig. 1;
- Fig. 7: shows a 3^{rd} view of a display of the inventive medical device as depicted in Fig. 1;
- Fig. 8: shows a 4^{th} view of a display of the inventive medical device as depicted in Fig. 1; and
- Fig. 9: shows a 5^{th} view of a display of the inventive medical device as depicted in Fig. 1;

The following paragraphs will describe various embodiments of the invention. For exemplary purpose only, the embodiments are outlined in relation to a medical device supporting health control and the respective method with regard to blood glucose level measurement. However, the used terminology and the description of the embodiments with respect to the medical device or health indicating method are not intended to limit the principles and ideas of the invention to such a single device or method and may be adapted to other physiological values accordingly.

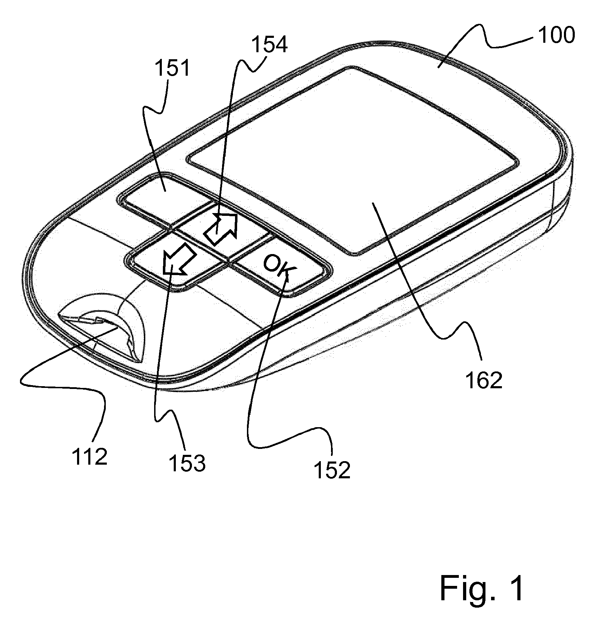

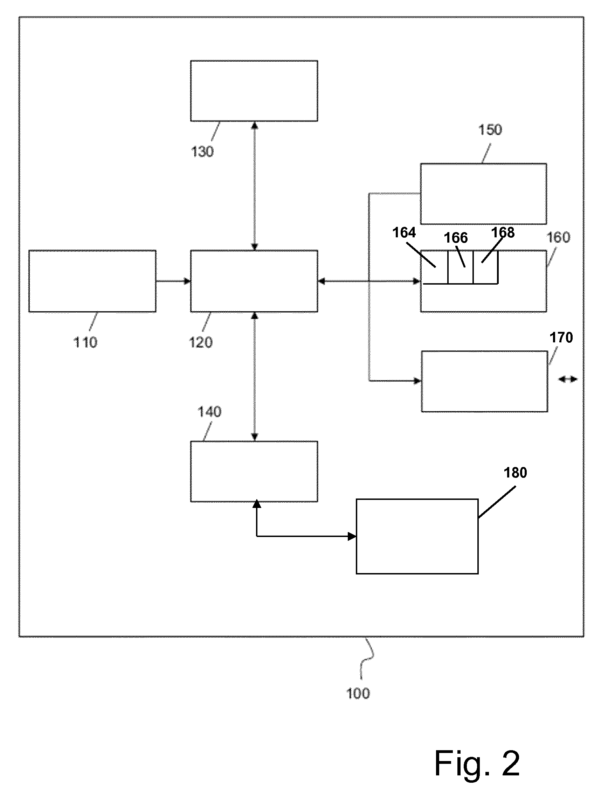

Fig. 1 is a schematic drawing and Fig. 2 is a schematic diagram of the medical device 100 according to a preferred embodiment of the invention. Preferably, the medical device 100 comprises a blood glucose measurement unit 110, which is arranged to measure the blood glucose level. Further, the measurement unit 110 comprises an interface and a slot 112 for inserting a test strip.

The blood glucose measurement unit 110 is connected to a receiving unit 120, which is arranged to forward e.g. blood glucose measurement data received from blood glucose measurement unit 110 to a data storage 130 (storage unit or means) or memory, such as a Flash memory. Alternatively, the receiving unit 120 may retrieve stored data such as e.g. blood glucose value data from the storage 130 and forward it to a processor 140 (processing unit or means), such as a microcontroller or microprocessor, a digital signal processor, and / or the like. Alternatively, the receiving unit 120 directly forwards the blood glucose value data received from the blood glucose measurement unit 110 to the processor 140.

Receiving unit 120 is further connected to a user input unit 150 of a user interface. The user input unit 150 is arranged to receive input from the user of the medical device 100 for example by key 151, confirmation key (OK button) 152, key 153 for scrolling down (downward button) and key 154 for scrolling up (upward button). The user input data are forwarded from the user input unit 150 to the receiving unit 120, which either forwards it to the processor 140 or to the data storage 130.

Furthermore, the user interface of medical device 100 comprises a display unit 160 with a display 162, which is connected to the receiving unit 120 as well. Preferably, the display unit 160 receives data to be displayed by the display 162 from the receiving unit 120 or the processor 140.

Preferably, the medical device 100 additionally comprises a further interface 170, for example a wired interface such as a serial port, a Universal Serial Bus (USB) interface, a mini-USB interface, or a wireless interface such as an infrared (e.g. an IRDA) interface, a Bluetooth™ interface, and / or the like, in order to receive data and / or to transmit data. The interface 170 is preferably connected to the receiving unit 120 in order to receive data from the receiving unit 120 and / or to forward data to the receiving unit 120.

Additionally, the medical device 100 comprises a clock unit 180 which provides a date and time information, preferably based on a clock generator, which may be displayed at the display 162. Further, the clock unit 180 provides date and time information in particular for generating a time stamp for an associated blood glucose measurement.

As outlined above, the medical device 100 preferably comprises a blood glucose measurement unit 110. Preferably, the blood glucose measurement unit 110 is arranged to measure the blood glucose level in the blood of e.g. the user by testing a drop of blood on a test strip that is inserted into the slot 112. The measurement may be made by e.g. an electrochemical method or an optical method. Full insertion of the test strip in the slot 112 may be detected by a respective sensor. The measured blood glucose value is transformed to blood glucose value data and forwarded preferably immediately or on demand to the receiving unit 120. Alternatively, the blood glucose measurement unit 110 is arranged to measure the blood glucose level of the user via infrared diagnosis or an alternative contactless measurement method.

According to a further alternative (not depicted in Fig. 1) the blood glucose measurement unit 110 is implanted in the body of the user of the medical device and forwards the data to the receiving unit 120 either via a wired connection or via a wireless connection. In an embodiment, such an implanted blood glucose measurement unit 110 is a continuous measurement sensor e.g. based on a chip which may allow a continuous closed loop control. In the latter case the medical device comprises two parts, one part contains the measurement unit 110 and the other part the remaining units of the medical device. The blood glucose measurement unit 110 preferably forwards the blood glucose measurement value data to the receiving unit 120 via interface 170. According to a further alternative the medical device does not comprise a blood glucose measurement unit which measures the blood glucose values, but receives blood glucose value data from an external unit.

The measurement of the blood glucose measurement is preferably triggered by the receiving unit 120 which sends a respective signal to the blood glucose measurement unit 110. According to one preferred alternative the receiving unit 120 receives a trigger signal generated by user input which is received via user input unit 150 or based on a signal from the slot 112 detecting a test strip. Alternatively, the trigger signal is generated automatically by the clock unit 180 or by the processor 140.

Preferably, the receiving unit 120 is represented e.g. by the input ports and output ports of a microprocessor or a bus system managing the data handling between several functional units. This includes bus systems, such as e.g. Advanced Microprocessor Bus Architecture bus systems implemented in a microprocessor or external bus systems connected to a microprocessor. Via the receiving unit 120, data are retrieved from the data storage 130 on demand and forwarded to the processor 140, to the display unit 160 or to the interface 170. Moreover, the receiving unit 120 forwards control signals, such as trigger signals or control signals e.g. to the blood glucose measurement unit 110, the display unit 160 or the interface 170.

The data storage 130 is arranged to store data entered via the user input unit 150, a plurality of blood glucose measurement data received from the blood glucose measurement unit 110 together with the time stamp and / or at least one event tag associated to each measurement data, data calculated from the plurality of blood glucose measurement values processed by the processor 140 and / or data received via interface 170.

Furthermore, data storage 130 is arranged to provide the stored data to the processor 140, to the display unit 160 and / or to the interface 170. The data storage 130 is preferably implemented as a semiconductor memory such as a Flash memory. Alternatively, it is implemented as a hard disk memory or an on-chip memory of the processor 140.

The processor 140 is preferably a microprocessor or any other functional unit capable of processing data.

The user input unit 150 is preferably implemented as a keyboard comprising one or more push buttons 151, 152, 153, 154. The keyboard may comprise one or more soft keys, wherein the function of the soft keys may be displayed on the display 162. Alternatively, the user input unit 150 is a key board or a touch screen. Alternatively, the user input unit 150 comprises a microphone for receiving speech input so that data can be entered via speech input.

After a blood glucose measurement a tag may be associated to the measurement value referring to lifestyle data by pressing the up or down keys 153, 154 scrolling upwards or downwards through the different tags which are for example the fasting tag, pre-meal tag, post-meal tag and no-tag respectively referring to a measurement value which is a fasting blood glucose value, a pre-meal blood glucose value, a post-meal blood glucose value and a blood glucose value that cannot be associated to one of the previous lifestyle parameter.

The display unit 160 preferably comprises an LCD or LED display 162. Preferably, the display displays a number of alphanumerical characters so that e.g. the presently measured blood glucose value can be displayed together with additional information or instructions for the user. Alternatively or additionally, the display unit 160 comprises a graphic display in order to display graphs or graphics such as icons. Further the display of the display unit 160 may comprise a touchscreen.

The display unit 160 further may also comprise a trend indicator 164 (trend indicating unit or means) which is adapted to indicate different trend categories, for example an increasing trend, a steady trend and a decreasing trend, symbolized e.g. as an arrow pointing up, an horizontal arrow and an arrow pointing down, respectively, at the display 162 (see Fig. 2).

Additionally, the display unit 160 comprises a color indicator 166 and a symbol indicator 168. The color indicator 166 is adapted to indicate an area with one of different colors on the display 162, for example an area of red color, an area of yellow color and an area of green color. The symbol indicator 168 is adapted to indicate one of different graphical symbol pictures on the display 162, for example an exclamation mark, a minus mark and a check mark.

The interface 170 is preferably a wireless interface, such as IRDA, Bluetooth™, GSM, UMTS, ZigBee, or WI-FI, etc. Alternatively, the interface is a wired interface, such as a USB port, mini-USB port, serial data port, parallel data port, etc., for receiving and transmitting data. In a further alternative embodiment the medical device 100 does not comprise an interface 170.

According to another alternative embodiment, medical device 100 comprises a memory card reader or a memory card reader interface. The memory card reader is preferably adapted to read information from a memory card, such as a Flash memory card, or any type of SIM card. For this purpose, the memory card comprises a memory, wherein at least one algorithm together with corresponding parameters, a history of the blood glucose values and / or insulin doses administered, etc. is stored. Thus, in the case that the medical device 100 has a defect, the relevant data may still be stored on the memory card which can be easily removed from the memory card reader of the medical device 100 and transferred to a new medical device 100. Moreover, the memory card 100 may be used in order to provide information on the history of the treatment to e.g. an HCP.

In the case that the memory card is a SIM card providing subscriber identification for a mobile communication network and the interface unit 170 is additionally a mobile communication interface, additional functions of the medical device 100 can be unlocked by the provider of the SIM card via a telecommunication channel. This offers the possibility that the medical device 100 can communicate with other telecommunication devices via predefined channels, such as UMTS or GSM. Via the international mobile subscriber identity, also called IMSI, stored in the SIM card, the medical device 100 identifies itself within the network and, thus, can be addressed via the network. In such a case the medical device 100 can be easily checked, remote controlled, updated, monitored, etc., via interface unit 170, e.g. by addressing the mobile communication unit with a phone number.

Furthermore, the medical device 100 is able to transmit data via SMS, e-mail or via mobile internet connection. Moreover, this offers the possibility to locate the medical device 100 in an emergency case.

In the case that the blood glucose measurement unit 110 is a continuous sensor which is e.g. implanted a dose delivery unit with an insulin pump forming an automatic delivery system may be additionally provided.

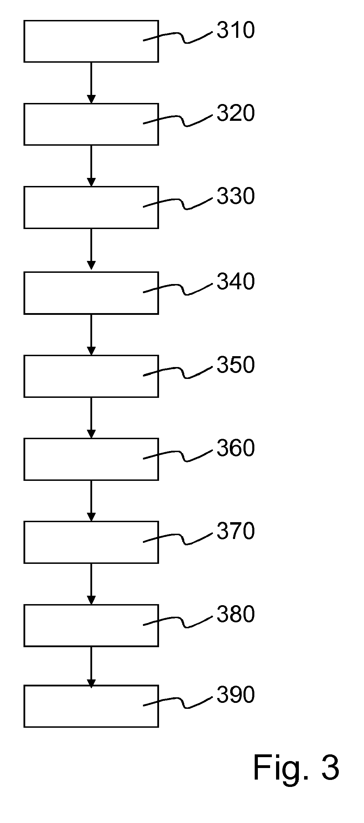

As shown in Fig. 3, the medical device 100 is capable to perform a number of operating processes. According to a preferred alternative after switching on, e.g. by pressing a key 151, 152, 153 or 154, preferably the confirmation key 152 for a predetermined time, or detection of a test strip within the slot 112, the medical device 100 performs initialization step 310 for initializing the functional components of the medical device 100. After this, the different operation modes which are implemented in the medical device 100, are displayed in the display step 320, preferably operation modes such as "Measure BG" and "Data Management and Analysis".

In step 330 the user selects one of the displayed operation modes via the user input unit 150, for example by means of the keys 153, 154 for scrolling down or up, and confirms the selection using the confirmation key 152.

In step 340 the selected operation mode is executed. As an example the mode "Measure BG" is selected for executing a blood glucose measurement. Upon execution of this mode the user / patient is requested to provide a test strip with a blood sample.

In the "Data Management and Analysis" mode the history of previous measurements and statistical results may be calculated and displayed. Further this mode allows the user to define and change some parameters of the medical device 100.

After selecting the mode "Measure BG", in step 350 a drop of blood is applied to the test portion of the test strip which is inserted in slot 112 of the medical device 100.

According to an alternative version of the operation process steps 310 to 340 may be skipped in the case that a specific operation mode is preselected. In this case, after initialization, the preselected operation mode, which is either preselected by the user or automatically selected in accordance with a specific event, for example the detection of a fully inserted test strip in slot 112, the operating process proceeds with the following step 350 and asks the user to apply a drop of blood. In step 360 it executes the preselected one or more operation modes, for example the mode "Measure BG".

Now in step 360 the measurement unit 110 determines e. g. by an electrochemical method the blood glucose level and displays the respective measurement value at the display 162. In the next step 370 the clock unit 180 generates the time stamp of the present measurement comprising a date and time information. The time stamp is also displayed in display 162 and both, the present blood glucose measurement value and the associated time stamp is transferred by receiving unit 120 to the data storage 130.

In the next step 380 an associated event tag may be automatically chosen by the processor and displayed in the display 162. The user may confirm this tag or select another tag and confirm this one by the confirmation key 152.

In the next optional step 390 a comment to the present measurement value may be selected by the user using the up and down keys 153, 154. The comment may then be confirmed with the confirmation key 152, wherein the chosen comment is then stored in the data storage 130 associated to the present measurement value as well.

When the medical device 100 is in the "Measure BG" mode, the device may turn into the sleep state automatically after for example 120 seconds without any new action. Once the device has returned a new measurement value, the device turns to the sleep state automatically after for example 60 seconds without any user interaction.

As explained above the medical device 100 provides at least one memory review mode which is called "Data Management and Analysis" mode. The respective display and calculations are explained in the following.

As mentioned above in an example embodiment the medical device 100 may be realized as a two-part device, wherein the first part comprises the measurement unit 110 and the second part comprises the data storage 130, the receiving unit 120, the processor 140, the user input unit 150, the display unit 160 with the trend indicator 164, the interface unit 170, and the clock unit 180. The inventive method is realized as a software program (application or "app") to run on the aforementioned hardware of a mobile device such as a smartphone or of another computer. The keys 151, 152, 153 and 154 may realized in this case as keys of a computer keyboard, keys on a mobile device such as a smartphone, or button fields on the display of a touchscreen of such a device. However, it is referred to a single-part medical device in the following description.

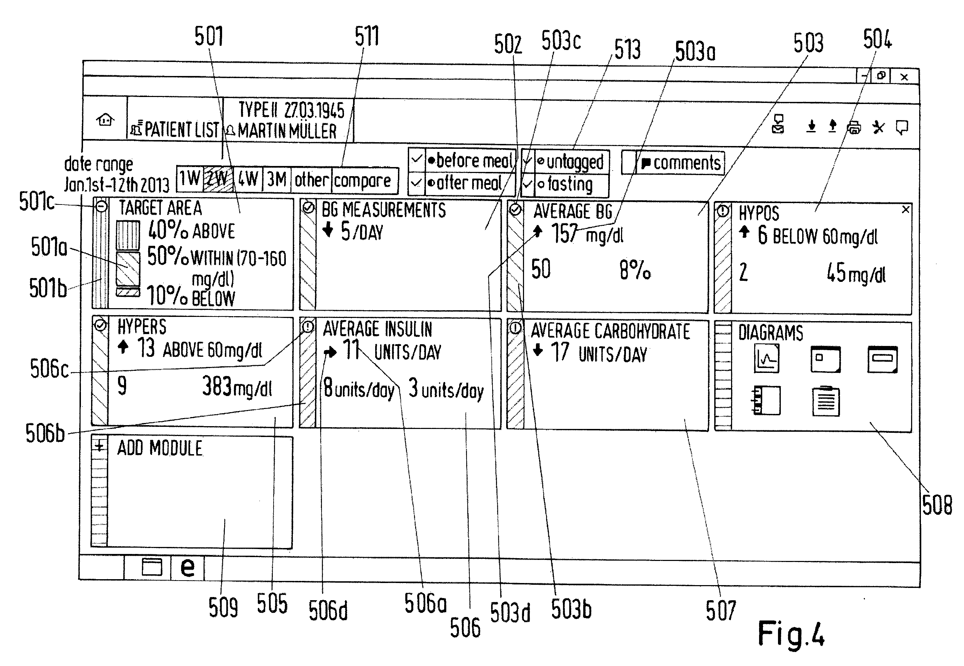

The "Data Management and Analysis" mode is entered when the user activates the medical device 100 by pressing e.g. the confirmation button 152. Then a display as depicted in Fig. 4 is shown.

Fig. 4 shows an exemplary view of a display 162 of the inventive data management unit comprising nine rectangular tiles 501, 502, 503, 504, 505, 506, 507, 508 and 509, each with a bar-like colored area at the left side edge. Further in the upper line of the display 162 there are several tabs 511 which give the user / patient the possibility to choose the selected date range for which the derived data in the tiles 501 to 508 are displayed, wherein the tab "1W" refers to one week, the tab "2W" refers to two weeks, the tab "4W" refers to four weeks, the tab "3M" refers to three months, the tab "other" refers to another date range individually selectable by the user / patient and the tab "compare" provides the possibility to compare data from the previous period with another period. For other languages, the labels may differ.

On the right hand side from the tabs 511 the tags and comments used with the measurement values are shown in rectangular tiles 513. Therein the tiles are marked with a check mark which were used in the data analysis, namely the tags "before meal", "after meal", "untagged" and "fasting" (or corresponding tags in other languages). Further, the possibility to provide comments to the single measurement values seems to be not used in the displayed case as this box is not provided with a check mark.

For example, the tile 503 shows as a derived, statistical value, namely the mean (average) blood glucose measurement value 503a (see title of this tile 503 "AVERAGE BG", wherein BG stands for blood glucose measurement value) of all measurement values of last two weeks in form of a numerical value and the respective unit (mg/dl), because the tab 511 "2W" is chosen and displayed in a darker color. Accordingly, all values shown in the display of Fig. 4 refer to last two weeks.

On the right hand side below the derived value 503a the tile displays another derived value, namely the estimated HbA1 c value (8 %) and the glucose variability (50). The glucose variability may be calculated as the standard deviation of a given number of glucose values, for example the glucose values from the given time range (two weeks in the example above) that are used for the average calculation.

The tile 503 further comprises on the left hand side a colored bar-like area 503b (color coding) with green color provided by the color indicator 166 and within the upper part of this colored area 503b a graphical symbol picture 503c showing an encircled check mark provided by the symbol indicator 168. The colored area 503b and the encircled check mark 503c contain the identical information for the user / patient that the average blood glucose value lies within the target area, which is for example defined by the HCP between 70 mg/dl and 160 mg/dl.

From the colored area 503b and the graphical symbol picture 503c showing a check mark the user / patient easily understands that the average blood glucose value within last two weeks is within the "good" range. The check mark 503c repeats the color information for color-blind people and intensifies the information "good" for all users.

If in another case, for example the average blood glucose value (e.g. determined as the arithmetic mean of all blood glucose values measurement values within the chosen time range of last 2 weeks) is below target, e.g. below 70 mg/dl, the colored area 503b would show red color and an encircled exclamation mark as a graphical symbol picture 503c. The colored area 503b with the same color and the same graphical symbol picture 503c would be shown if the average blood glucose value is above the target range. The association of the color of the colored area 503b or the graphical symbol picture 503c, respectively, and the average blood glucose value may be provided by a matrix containing the blood glucose value ranges and the respective colors or graphical symbol icons or pictures.

On the left hand side of the derived value 503a an arrow 503d pointing up is shown provided by the trend indicator 164. This arrow 503d displays the trend information with regard to the derived value 503a and tells the user / patient that the trend of the blood glucose measurement values within the chosen time range of two weeks is increasing.

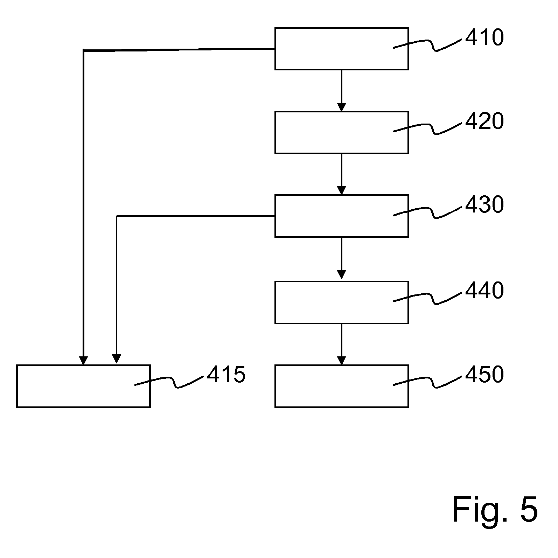

With regard to a fasting blood glucose measurement value the trend may be for example calculated using the method depicted in Fig. 5.

For each fasting measurement value the processor 140 selects in step 410 via the receiving unit 120 from the data storage 130 a second group of measurement values containing the fasting tag of the recent e.g. three days, for example the fasting measurement values of the present day and the two days before (days 0, -1 and -2), wherein at least two fasting measurement values within the three day period must be available. If less than two fasting measurement values, i.e. only one fasting measurement value, within the three day period is available the procedure moves to step 415 and the trend indicator 164 does not calculate and display a respective trend arrow and the display 162 stays empty in the upper left corner or shows an error sign.

Then, in step 420 the arithmetic mean value is determined from these 3 or 2 fasting measurement values as the second mean value CURR_FAST_AVG.

Further in step 430, the processor 140 selects from the data storage 130 via the receiving unit 120 a first group of measurement values based on the associated time stamp comprising seven days before the recent three days, i.e. three days to nine days prior the recent fasting measurement value (days -3 to -9), wherein at least a first number limit of for example 5 fasting measurement values within the seven day period must be available. Otherwise, the procedure moves to step 415 and the processor 140 does not calculate a trend and the trend indicator 164 does not display any trend arrow or shows an error sign.

In step 440, if there are enough fasting measurement values, from the fasting measurement values of the first group the median is determined as a first mean value PAST_FAST_MED.

Now, in the next step 450 the trend is calculated and displayed using respective arrows in the following way.

If the second mean value CURR_FAST_AVG is greater than a low limit of 125 mg/dl and the difference between the second mean value and the first mean value is greater than 20% of the second mean value (corresponding to a relative tolerance range), i.e. (CURR_FAST_AVG - PAST_FAST_MED) > (CURR_FAST_AVG * 20%),
the fasting trend up arrow is displayed on the display 162.

Also, the fasting trend up arrow is displayed on the display 162 if the second mean value CURR_FAST_AVG is less than or equal the low limit of 125 mg/dl and the difference between the second mean value and the first mean value is greater than 25 mg/dl (corresponding to an absolute tolerance range), i.e.
(CURR_FAST_AVG - PAST_FAST_MED) > 25 mg/dl.

If the second mean value CURR_FAST_AVG is greater than the low limit 125 mg/dl and the difference between the first mean value and the second mean value is greater than 20% of the second mean value (corresponding to the relative tolerance range), i.e.
(PAST_FAST_MED - CURR_FAST AVG) > (CURR_FAST_AVG * 20%),
the fasting trend down arrow 202 is displayed on the display 162.

Also, the fasting trend down arrow 202 is displayed on the display 162 if the second mean value CURR_FAST_AVG is less than or equal the low limit of 125 mg/dl and the difference between the first mean value and the second mean value is greater than 25 mg/dl (corresponding to an absolute tolerance range), i.e.
(PAST_FAST_MED - CURR_FAST_AVG) > 25 mg/dl.

If there is a valid calculation, i.e. at least two fasting measurement values in the second group and at least 5 fasting measurement values in the first group of measurement values, in all other cases the fasting trend steady arrow 201 is displayed by the trend indicator 164 at the display 162.

The fasting trend arrows showing in upward, downward or horizontal direction are easy to understand for the patient and provide a reliable and descriptive assessment of the fasting blood glucose value development over a time range of about 1.5 weeks from the respective fasting value. Similarly, the trend of other derived values may be determined and shown using a trend arrow at the display 162, in particular within the respective tile of the derived value.

The tile 501 in Fig. 4 contains as a derived value 501 a a distribution of blood glucose measurement values within last two weeks in a bar chart providing e.g. the information that 50% of the blood glucose measurement values within last two weeks were determined as within the target range of 70 mg/dl to 160 mg/dl, 40% of the blood glucose measurement values were above this target and 10% below this target. The bar-like colored area 501 b on the left hand side of this tile 501 provided by the color indicator 166 shows an orange color symbolizing that the distribution of the blood glucose measurement values is in between good and bad. Accordingly, the graphical symbol picture 501 c on the upper part of the colored area 501 b provided by the symbol indicator 168 shows an encircled minus mark. A trend value is not displayed on the first tile 501.

In an embodiment there will be different ranges for differently tagged glucose readings, e. g. fasting reading ranges may be different to pre- or post-meal ranges.

The sixth tile 506 refers to the derived value 506a of average administered insulin units per day within this time range and displays a value of 11 units per day which is a low value so that the colored area 506b provided by the color indicator 166 shows a red color. Accordingly, the graphical symbol picture 506c provided by the symbol indicator 168 shows an encircled exclamation mark. The administered insulin units may be entered into the medical device 100 using the input unit. According to the trend arrow 506d pointing horizontally to the right side provided by the trend indicator this derived value 506a has a steady trend.

The association of the color of the colored area or the graphical symbol picture to the respective derived value and / or rating value and / or trend value may be derived from a respective matrix containing the pre-determined ranges of the aforementioned values and the associated color or graphical symbol picture. The matrix may be stored in the data storage 130.

All tiles 501 to 509 are rearrangeable and configurable, also with regard to whether they are visible or not. Additionally, tiles with critical information of the patient status, in particular tiles which show the red color in the colored area may be automatically arranged in the way that they always stay on top of the list of tiles 501 to 509.

Each tile 501 to 509 is adapted to "flip over" if it is double clicked allowing the HCP to change the settings for this particular patient and / or this particular tile.

By using the last tile 509 entitled "ADD MODULE" the user / patient may add another tile by an according definition of the settings.

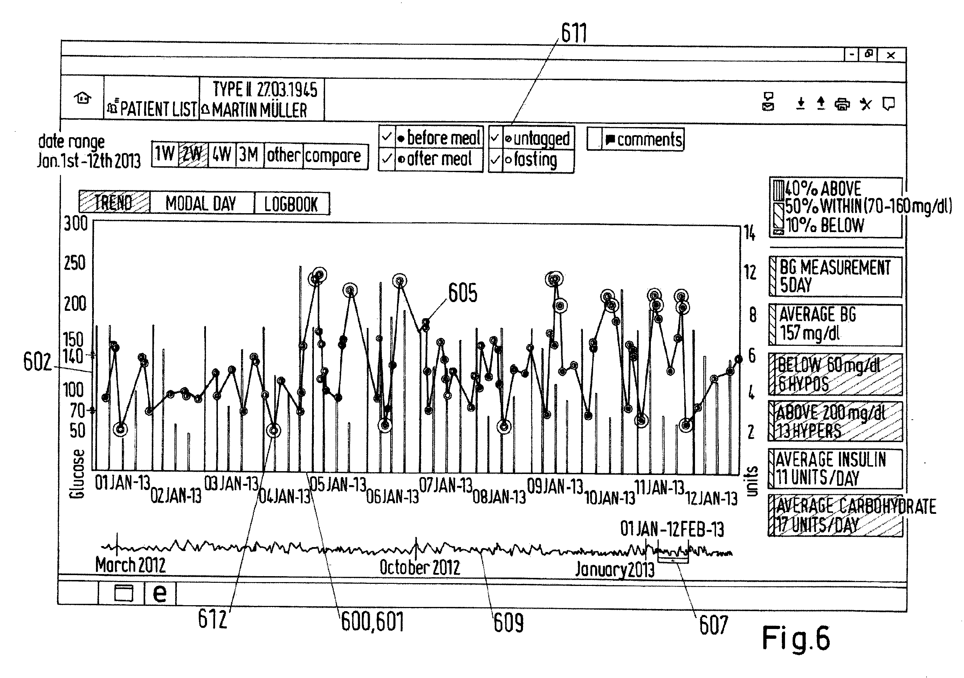

Single clicking on any tile 501 to 508 gets the user to the respective trend chart 600 which is depicted in Fig. 6. Therein, each measurement value of this time range (here two weeks) is plotted as a point in a diagram providing on the x-axis 601 the time and date of the measurement (derived from the time stamp of the respective measurement value) and on the y-axis 602 the blood glucose level. The measurement point further contains, if applicable, a symbol 605 showing the kind of measurement regarding the associated tag as depicted in the tiles 611 in the upper line of the display.

The consecutive measurement points are connected with a line to make the user understand the ups and downs of the blood sugar level within the chosen time range.

In the trend chart 600 the hypos or hypers are emphasized by a colored ring 612 appearing like a shadow around the symbol 605, if applicable, referring to the tag of this measurement point. The colored ring 612 emphasizes and highlights the respective measurement point and has the advantage that the user / patient may easily derive the distribution of the hypos and hypers from the chart 600.

Using the slider 607 of the "history" time scale 609 depicted in the lower line the user / patient may choose which period (time section) he or she wishes to look at from a, for example, 3 months line. This slider forms a section of the blood glucose values line, which helps the user / patient to see where there is data (if the patient does not test often enough), and get a first glimpse of where problems may lie (for example with regard to too much variability).

On the right hand side of the trend chart a "mini-dashboard" shows some important statistical values similar to the tiles view of Fig. 4.

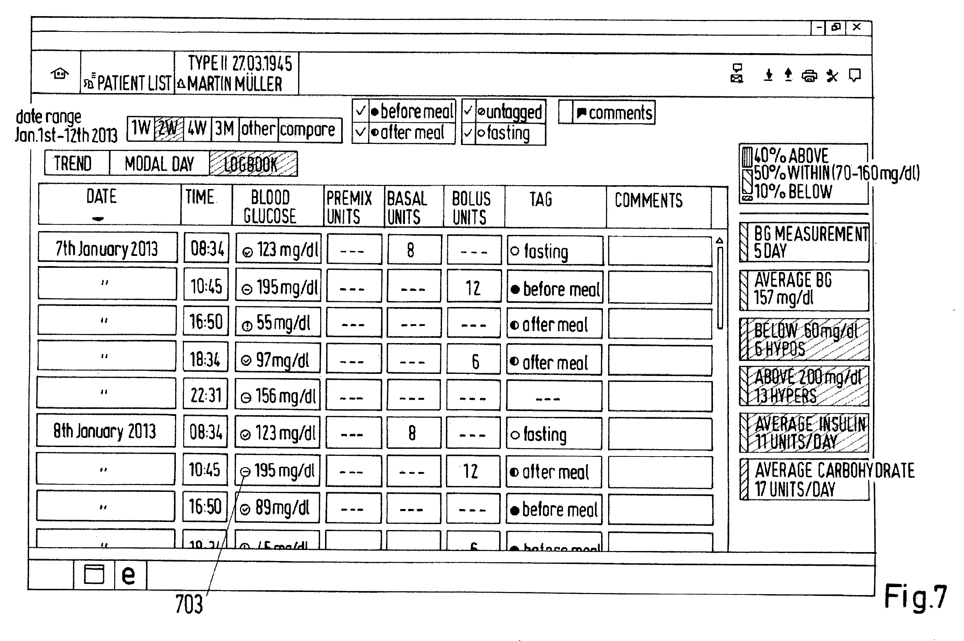

Using the respective tab "LOGBOOK" above the chart 600 the user may switch to the associated logbook note which is depicted in Fig. 7. In this view each single measurement value (column "BLOOD GLUCOSE") within the chosen time range (here two weeks) is listed comprising the associated time stamp (columns "DATE" and "TIME"), the respective tag (column "TAG") and comment (column "COMMENT"). Additionally, the associated administered dose is listed (columns "PREMIX UNITS", "BASAL UNITS" and "BOLUS UNITS"). Additional columns showing other data may be added, for example the ingested carbohydrate value.

Further, each blood glucose measurement value is accommodated with the graphical symbol picture 703 as it is used in the view with the tiles depicted in Fig. 4 (check mark, minus mark, exclamation mark) showing whether the blood glucose value is within the target range specified by the HCP. Additionally, the graphical symbol picture 703 contains the corresponding color of the colored area as used in the tiles view of Fig. 4 which promotes the understanding of the assessment of the logbook note.

On the right hand side of the "LOGBOOK" display a "mini-dashboard" shows some important statistical values similar to the tiles view of Fig. 4.

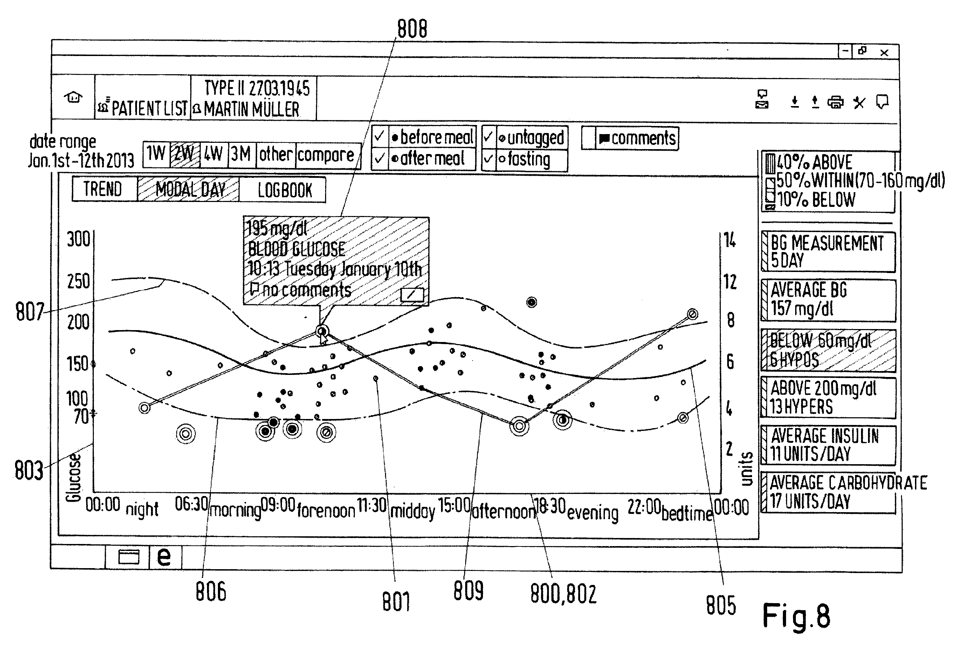

Using the tab "MODAL DAY" the user / patient may switch to a chart 800 depicted in Fig. 8. This chart shows the distribution of the single blood glucose measurement values 801 of the chosen time range (here two weeks) over a 24 hour day. The x-axis 802 refers to the time and the y-axis 803 of the chart 800 refers to the blood glucose level. Additionally, it shows the average value (line 805), for example the 25th percentile (line 806) and 75th percentile (line 807) of the measurement values. Clicking on a data point provides the patient/user with the particulars of this data point in form of a flag 808 and the consecutive measurement values of the respective single day are connected with a line 809. Similar to the logbook mode a "mini dashboard" with some important statistical values is shown on the right hand side of the chart 800.

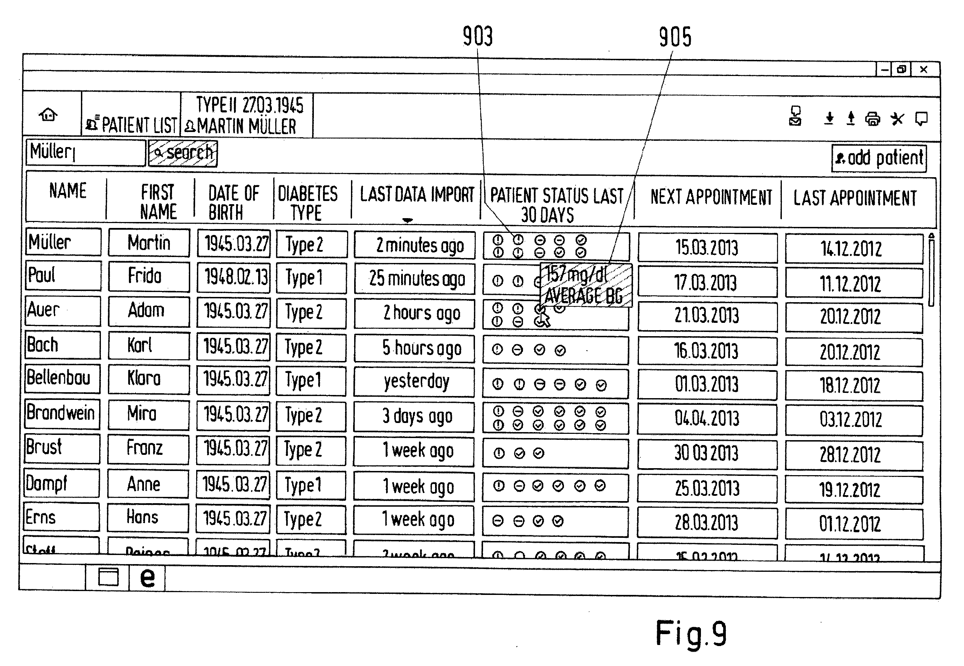

Fig. 9 shows a patient list which may be configured such that it is only accessible for an HCP, not by a patient. The display arrangements of Figs. 4 and 6 to 8 may also be accessible for the HCP.

The patient list shows the name of the patient, date of birth, diabetes type, the date of last data import as well as in the last two columns the date of the last appointment with the HCP and the date of the next appointment with the HCP. In a column "PATIENT STATUS LAST 30 DAYS" in the center of the list for each patient a status of last thirty days is provided showing several graphical symbol pictures analogous to the graphical symbol pictures of the tiles view of Fig. 4. By clicking on one of these status symbols more information with regard to this status is provided using a flag 905. From this, the HCP may easily judge which patient probably needs an earlier appointment or which treatment develops in a good, bad or stable condition.

The patient list may provide an automatic sorting by "PATIENT STATUS", so that the patients with the most critical condition will be shown on top of the list.

If the HCP clicks on a line containing the information "add patient" a screen for patient data input opens. Therein the HCP may include patient details comprising e-mail address and a field to activate access to this program and method for the patient.

## Claims

**1.** A data management unit for supporting health control comprising
• a display (162),
• a processor (140) adapted to receive a user-selectable group of measurement values of a plurality of measurement values of a physiological parameter, for example a blood glucose level, to calculate at least one derived value (501 a, 503a, 506a) from the group of measurement values and to determine for each derived value (501 a, 503a, 506a) a corresponding rating value and / or a corresponding trend value,
• a trend indicator (164), wherein the trend indicator is adapted to indicate different trend categories on the display (162), for example an increasing trend, a steady trend and a decreasing trend, as a respective graphical picture (503d),
• a color indicator (166), wherein the color indicator is adapted to indicate an area (501 b, 503b, 506b) with one of at least two different colors on the display (162), for example a red color and a green color,
wherein the display (162) is adapted to display the at least one derived value (501 a, 503a, 506a), each in a separate tile (501, 502, 503, 504, 505, 506, 507), as a numerical value and / or a graphical picture,
wherein the processor (140) interacts with the trend indicator (164) such that the trend indicator (164) displays within each tile (501, 502, 503, 504, 505, 506, 507) one of the different trend categories based at least in part on the corresponding trend value of the at least one derived value (501 a, 503a, 506a) of the respective tile (501, 502, 503, 504, 505, 506, 507) and / or
the processor (140) interacts with the color indicator (166) such that the color indicator displays within each tile one area (501 b, 503b, 506b) of one of the at least two different colors based at least in part
• on the at least one derived value (501 a, 503a, 506a) of the respective tile (501, 502, 503, 504, 505, 506, 507) and / or
• on the corresponding rating value of the at least one derived value (501 a, 503a, 506a) of the respective tile (501, 502, 503, 504, 505, 506, 507) and / or
• on the corresponding trend value of the at least one derived value (501 a, 503a, 506a) of the respective tile (501, 502, 503, 504, 505, 506, 507).

**2.** The data management unit according to claim 1, wherein the data management unit further comprises a symbol indicator (168), wherein the symbol indicator is adapted to indicate one of different graphical symbol pictures (503c, 703, 903) on the display (162), for example an exclamation mark and a check mark, wherein the processor interacts with the symbol indicator (168) such that the symbol indicator displays within each tile one of the different graphical symbol pictures based at least in part
• on the at least one derived value (501 a, 503a, 506a) of the respective tile (501, 502, 503, 504, 505, 506, 507) and / or
• on the corresponding rating value of the at least one derived value (501 a, 503a, 506a) of the respective tile (501, 502, 503, 504, 505, 506, 507) and / or
• on the corresponding trend value of the at least one derived value (501 a, 503a, 506a) of the respective tile (501, 502, 503, 504, 505, 506, 507).

**3.** The data management unit according to any of the preceding claims, wherein the derived value (501 a, 503a, 506a) is a mean value of the group of measurement values, a number of particular, pre-defined measurement values within the group of measurement values, a distribution of the measurement values of the group within predefined intervals, or an estimated value.

**4.** The data management unit according to any of the preceding claims, comprising a first matrix referring to different ranges of the at least one derived value (501 a, 503a, 506a) and / or to different ranges of the corresponding rating values and / or to different ranges of the corresponding trend values, wherein the first matrix assigns one of the at least two different colors to each range of aforementioned values or to each combination of ranges of aforementioned values.

**5.** The data management unit according to any of the claims 2 to 4, comprising a second matrix referring to different ranges of the at least one derived value and / or to different ranges of the corresponding rating values and / or to different ranges of the corresponding trend values, wherein the second matrix assigns one of the different graphical symbol pictures to each range of aforementioned values or to each combination of ranges of aforementioned values.

**6.** The data management unit according to any of the preceding claims, wherein the trend indicator (164) is adapted to show on the display (162) an up arrow (503d) as the increasing trend sign, a down arrow as the decreasing trend sign and a horizontal arrow as the steady trend sign.

**7.** The data management unit according to any of the preceding claims, wherein the data management unit is adapted to consider for each measurement value an associated event tag for user selection of the group of measurement values of the plurality of measurement values and / or for calculation of the derived value (501 a, 503a, 506a) from the group of measurement values.

**8.** A method for supporting health control for a data management unit (100) comprising the following steps:
• receiving a user-selectable group of measurement values of a plurality of measurement values of a physiological parameter, for example a blood glucose level,
• calculating at least one derived value (501 a, 503a, 506a) from the group of measurement values and determining for each derived value a corresponding rating value and / or a corresponding trend value,
• displaying on a display (162) the at least one derived value (501 a, 503a, 506a), each in a separate tile (501, 502, 503, 504, 505, 506, 507), as a numerical value and / or a graphical picture,
• indicating within each tile (501, 502, 503, 504, 505, 506, 507) one of different trend categories, for example an increasing trend, a steady trend and a decreasing trend, as a respective visible graphical picture (503d), based at least in part on the corresponding trend value of the at least one derived value (501 a, 503a, 506a) of the respective tile (501, 502, 503, 504, 505, 506, 507) and / or
• indicating within each tile (501, 502, 503, 504, 505, 506, 507) an area with one of at least two different colors, for example a red color and a green color, based at least in part
○ on the at least one derived value (501 a, 503a, 506a) of the respective tile (501, 502, 503, 504, 505, 506, 507) and / or
○ on the corresponding rating value of the at least one derived value (501 a, 503a, 506a) of the respective tile (501, 502, 503, 504, 505, 506, 507) and / or
○ on the corresponding trend value of the at least one derived value (501 a, 503a, 506a) of the respective tile (501, 502, 503, 504, 505, 506, 507).

**9.** The method according to claim 8, wherein the method comprises the additional step of indicating within each tile one of different graphical symbol pictures (501 c, 703, 903), for example an exclamation mark and a check mark, based at least in part
○ on the at least one derived value (501 a, 503a, 506a) of the respective tile (501, 502, 503, 504, 505, 506, 507) and / or
○ on the corresponding rating value of the at least one derived value (501 a, 503a, 506a) of the respective tile (501, 502, 503, 504, 505, 506, 507) and / or
○ on the corresponding trend value of the at least one derived value (501 a, 503a, 506a) of the respective tile (501, 502, 503, 504, 505, 506, 507).

**10.** The method according to any of the claims 8 to 9, wherein the derived value (501 a, 503a, 506a) is a mean value of the group of measurement values, a distribution of the measurement values of the group within predefined intervals, a number of particular, pre-defined measurement values within the group of measurement values, or an estimated value.

**11.** The method according to any of the claims 8 to 10, wherein the one of the at least two different colors is chosen from a first matrix referring to different ranges of the at least one derived value and / or to different ranges of the corresponding rating values and / or to different ranges of the corresponding trend values, wherein the first matrix assigns one of the at least two different colors to each range of aforementioned values or to each combination of ranges of aforementioned values.

**12.** The method according to any of the claims 9 to 11, wherein the one of the different graphical symbol pictures is chosen from a second matrix referring to different ranges of the at least one derived value and / or to different ranges of the corresponding rating values and / or to different ranges of the corresponding trend values, wherein the second matrix assigns one of the different graphical pictures to each range of aforementioned values or to each combination of ranges of aforementioned values.

**13.** The method according to any of the claims 8 to 12, wherein an up arrow (503d) as the increasing trend sign, a down arrow as the decreasing trend sign and a horizontal arrow as the steady trend sign is shown on the display (162).

**14.** The method according to any of the claims 8 to 13, wherein an associated event tag for each measurement value is considered for user selection of the group of measurement values of the plurality of measurement values and / or for calculation of the derived value (501 a, 503a, 506a) from the group of measurement values.

**15.** A computer program for supporting health control comprising:
• code for receiving a user-selectable group of measurement values of a plurality of measurement values of a physiological parameter, for example a blood glucose level,
• code for calculating at least one derived value (501 a, 503a, 506a) from the group of measurement values and determining for each derived value a corresponding rating value and / or a corresponding trend value,
• code for indicating on a display (162) the at least one derived value, each in a separate tile (501, 502, 503, 504, 505, 506, 507), as a numerical value and / or a graphical picture,
• code for indicating within each tile (501, 502, 503, 504, 505, 506, 507) one of different trend categories, for example an increasing trend, a steady trend and a decreasing trend, as a respective visible graphical picture, based at least in part on the corresponding trend value of the at least one derived value of the respective tile and / or
• code for indicating within each tile (501, 502, 503, 504, 505, 506, 507) an area (503b) with one of at least two different colors, for example a red color and a green color, based at least in part
○ on the at least one derived value of the respective tile (501, 502, 503, 504, 505, 506, 507) and / or
○ on the corresponding rating value of the at least one derived value (501 a, 503a, 506a) of the respective tile (501, 502, 503, 504, 505, 506, 507) and / or
○ on the corresponding trend value of the at least one derived value (501 a, 503a, 506a) of the respective tile (501, 502, 503, 504, 505, 506, 507).

**16.** The computer program according to claim 15 further comprising code for indication within each tile (501, 502, 503, 504, 505, 506, 507) one of different graphical symbol pictures (503c, 703, 903), for example an exclamation mark and a check mark, based at least in part
○ on the at least one derived value (501 a, 503a, 506a) of the respective tile (501, 502, 503, 504, 505, 506, 507) and / or
○ on the corresponding rating value of the at least one derived value (501 a, 503a, 506a) of the respective tile (501, 502, 503, 504, 505, 506, 507) and / or
○ on the corresponding trend value of the at least one derived value (501 a, 503a, 506a) of the respective tile (501, 502, 503, 504, 505, 506, 507).

**16.** A computer program product comprising a computer-readable medium bearing computer program code embodied therein for use with a computer, wherein the computer program code comprises the computer program according to any of the claims 15 or 16.

**17.** A medical device (100) for supporting health control, the device comprising the data management unit according to any of the claims 1 to 7.
